# EUROPÄISCHE PATENTSCHRIFT

(11) **EP 1 291 698 B1**
(45) Veröffentlichungstag und Bekanntmachung des Hinweises auf die Patenterteilung: **07.06.2006**
(21) Anmeldenummer: 02026641.7
(22) Anmeldetag: 27.03.1998
(51) Int. Cl.: G02B 23/24, A61B 1/00, G02B 7/04

(54) **Vorrichtung zum Positionieren von Bauelementen innerhalb endoskopischer Systeme**
Device for positioning components in endoscopic systems
Dispositif pour le positionnement d'éléments à l'intérieur de systèmes optiques

(30) Priorität: 29.03.1997 DE 19713276
(43) Veröffentlichungstag der Anmeldung: 12.03.2003
(62) Teilanmeldung aus: 98919177.0
(73) Patentinhaber: Karl Storz GmbH & Co. KG, 78532 Tuttlingen (DE)
(72) Erfinder: Kehr, Ulrich, 70771 Leinfelden-Echterdingen (DE); Rudischhauser, Jurgen, 78532 Tuttlingen (DE); Dahmen, Jan, 79606 Seitingen (DE)
(74) Vertreter: Heuckeroth, Volker

(56) Entgegenhaltungen:
- DE-A- 19 521 654
- DE-C- 970 298
- DE-U- 8 810 044
- US-A- 5 139 383
- US-A- 5 359 992
- US-A- 5 539 266

## Beschreibung

Die Erfindung betrifft eine Vorrichtung zum Positionieren von Bauelementen innerhalb endoskopischer Systeme, mit einem hermetisch dichten Gehäuse, mit einem um die Außenseite des Gehäuses drehbar angeordneten äußeren Ringelement, das umfänglich zumindest einen Magnet trägt, mit einem weiteren innerhalb des dichten Gehäuses angeordneten inneren Ringelement, das ebenfalls zumindest einen Magnet trägt, wobei die Magnete so ausgerichtet sind, dass eine Drehbewegung des äußeren Ringelementes über magnetischen Kraftschluss den zumindest einen inneren Magneten bewegt, wobei diese Bewegung zum Positionieren von Bauelementen dient.

Eine derartige Vorrichtung ist aus der DE 195 21 654 A1 bekannt.

Positionieren im Sinne der vorliegenden Erfindung beinhaltet ein axiales Verschieben und/oder ein umfängliches Verschieben und dadurch in Stellung Bringen von Bauelementen.

Der Begriff Bauelemente umfasst bspw. optische Bauelemente, z.B. Linsen in einem Optikkopf eines Endoskopes. Eine axiale Verschiebung der Systeme dient zum Fokussieren oder zum Scharfstellen des optischen Systems. Unter den Begriff Bauelemente fallen auch mechanische Bauelemente, die bspw. in ein optisches System ein- oder ausgeschwenkt werden sollen, wie bspw. Filter, Blenden oder dgl. Dabei kann die Handhabung der Positioniervorrichtung am proximalen Ende des endoskopischen Systems erfolgen, das zu bewegende Bauelement kann auch am distalen Ende angeordnet sein, und über ein Gestänge mit der Positioniervorrichtung verbunden sein.

Unter endoskopischen Systemen im Sinne der vorliegenden Erfindung werden Endoskope oder auch endoskopische Kamerasysteme verstanden.

Unter einem hermetisch dichten Gehäuse im Sinne der vorliegenden Erfindung versteht man ein derart abgeschlossenes Gehäuse, dass dieses bspw. autoklaviert werden kann, ohne dass die Gefahr besteht, dass bei den extremen Temperaturschwankungen in den Innenraum des Gehäuses Feuchtigkeit oder Flüssigkeiten, somit Kontaminationen eindringen können. Die Magnete des äußeren, um die Außenseite des dichten Gehäuses angeordneten Ringelements wirken mit den Magneten des innerhalb des dichten Gehäuses angeordneten Ringelements im Sinne einer Magnetkupplung.

Bei der eingangs genannten optischen Vorrichtung ist der innere Magnet in einer schraubenlinienförmigen Schlitzöffnung einer im Innern des dichten Gehäuses ortsfest angeordneten Hülse aufgenommen. Im Innern der Hülse ist axial verschiebbar und drehbar eine Fassung angeordnet, die Linsen einer Objektivgruppe trägt. Der als Rundmagnet ausgebildete innere Magnet greift in eine radiale Sacklochbohrung am Außenumfang der Fassung ein.

Der Magnet des äußeren drehbaren Ringelementes ist als Rechteckmagnet ausgebildet, der mit dem Rundmagneten in Wirkverbindung steht, also diesem in etwa gegenüberliegt.

Wird nunmehr das äußere Ringelement mit dem Rechteckmagneten gedreht, wird der innere Rundmagnet ebenfalls gedreht. Aufgrund der Tatsache, dass dieser in einem schraubenlinienförmigen Schlitz der Hülse aufgenommen ist, bewegt sich der Rundmagnet auch in axialer Richtung.

Demzufolge muss der am äußeren Ring angeordnete Rechteckmagnet eine solche axiale Erstreckung aufweisen, die dem maximalen axialen Laufweg des inneren Rundmagneten während dessen Lauf in dem schraubenlinienförmigen Schlitz in der Hülse entspricht.

Dadurch, dass der Rundmagnet in einer radialen Sacklochbohrung an der Außenseite der Fassung in Eingriff steht, wird diese entsprechend der axialen Vorschubbewegung des Rundmagneten axial verschoben.

Diese Ausgestaltung hat den Nachteil, dass aufgrund der axialen Beweglichkeit des inneren Magnetes der äußere Ringmagnet eine sehr lange axiale Erstreckung aufweisen muss, somit groß baut, was zu sperrigen und großen Bauweisen führt. Aufgrund der Tatsache, dass der Magnet im äußeren Ring ein relativ großer Magnet ist, streut dieser in Richtung Außenseite Magnetfelder aus, die Störungen an solchen Systemen ausüben können, die mit Elektronenstrahlungen arbeiten, also bspw. Monitore von endoskopischen Kameras oder dgl. Darüber hinaus steht der innere Rundmagnet mechanisch mit zwei unterschiedlichen Bauelementen in Verbindung, nämlich der äußeren ortsfesten Hülse mit dem schraubenlinienförmigen Schlitz und der Sacklochbohrung der inneren Fassung des optischen Systems. Um ein verklemmfreies Laufen zu gewährleisten, müssen exakt bearbeitete Teile und auch ein bestimmte Spiel vorhanden sein, so dass, insbesondere bei Umkehrbewegungen, ruckartige Bewegungen mit Verstellungen des optischen Systems verbunden sind.

Aus der US-A-5 359 992 ist eine Vorrichtung zum Positionieren von Bauelementen innerhalb endoskopischer Systeme bekannt, bei dem im äußeren Ringelement diametral gegenüberliegend zwei schraubenlinienförmige Schlitze ausgebildet sind, in denen jeweils diametral gegenüberliegend Rundmagnete eingesetzt sind. Die Rundmagnete greifen in eine axial verlaufende Aussparung an der Außenseite einer in dem Ring angeordneten Hülse an. Ein Drehen des äußeren Ringes verursacht somit eine Axialverschiebung des äußeren Magnetes. Im inneren abgeschlossenen Bereich sind entsprechend diametral gegenüberliegende Magnete vorhanden, die den Bewegungen des äußeren Magnetes folgen und somit die Kupplung bewirken. Das innere Stellelement mit den inneren Magneten ist mechanisch nicht geführt, so dass, falls das innere Element aufgrund eines Schockes oder eines Stoßes aus dem magnetischen Feld des äußeren Magnetes gelangt, es im Innenraum des Gehäuses hin- und herbewegbar und verdrehbar ist. Um die Funktionsweise zu erhalten, muss zunächst das Endoskop so verschwenkt werden, bis die äußeren und inneren Magnete wieder in Wirkverbindung stehen.

Eine ähnliche Konstruktion ist aus dem DE 88 10 044 U1 bekannt, bei dem ein an der Innenseite des äußeren Ringes angeordneter Magnet mit einem sehr langerstreckten inneren Magnet in Verbindung steht.

Der äußere Magnet wird axial bewegt und zieht damit den inneren Magnet mit und bewirkt dadurch die Kupplung.

Ein Fernrohr mit diesem Grundprinzip der Objektivverstellung ist auch aus der deutschen Patentschrift 970 298 bekannt. Auch hier ist möglich, dass bei mechanischen Schocks der innere Magnet außer magnetischem Eingriff mit dem äußeren Magnet kommt.

Es ist Aufgabe der vorliegenden Erfindung, eine Vorrichtung der eingangs genannten Art dahingehend zu verbessern, dass bei kleiner Bauweise eine effektive Positionierung von Bauelementen auf Dauer funktionssicher durchführbar ist.

Erfindungsgemäß wird die Aufgabe dadurch gelöst, dass sowohl das äußere als auch das innere Ringelement als drehbare, jedoch axial unverschieblich angeordnete Ringe ausgebildet sind, die gegenüberliegend die Magnete tragen, und dass der innere Ring mit den zu positionierenden Bauelementen mechanisch in Verbindung steht, wobei der innere Ring Bauelemente trägt, die bei einem Verdrehen des inneren Rings in ein optisches System ein- bzw. ausschwenkbar sind.

Es wird nun von dem Prinzip abgewichen, dass zumindest einer oder gar beide Magnete axial beweglich sind.

Es sind nunmehr nur noch verdrehbare, die Magnete tragende Ringe vorhanden, so dass die Axialerstreckung der Magnetkupplung sehr kurz ist. Dadurch baut die Magnetkupplung nicht nur kurz, sondern es ist kein großflächiger axialer Bereich vorhanden, über den störende Magnetfelder abgestrahlt werden können, es kann somit effektiv die störende Strahlung, d.h. ohne große ausladende Baumaßnahmen, abgeschirmt werden.

Die Relativstellung zwischen innerem und äußerem Magnet ändert sich beim Verdrehen nicht. Wird der äußere Ring mit dem äußeren Magnet gedreht, dreht sich entsprechend der innere Ring mit dem inneren Magnet. Es fallen somit schraubenlinienförmige Führungen für bewegte Magnete völlig weg. Die Umsetzung der Drehbewegung des inneren Ringes wird über die mechanische Verbindung zwischen den positionierenden Bauelementen und dem Ring bewerkstelligt.

Weiterhin erfindungsgemäß trägt der innere Ring Bauelemente, die bei einem Verdrehen des inneren Ringes in ein optisches System ein- bzw. ausschwenkbar sind.

Dies hat den erheblichen Vorteil, dass über die Magnetkupplung in ein optisches System seitlich Bauelemente wie Filter, Gitter, Blenden oder dgl. ein- bzw. ausgeschwenkt werden können.

In diesem Fall fällt dann die Drehachse der Ringe nicht mit der optischen Achse zusammen. Die Bauelemente werden wie in einer Art eines Revolvermagazins in das optische Systeme ein- bzw. ausgeschwenkt.

Insgesamt gesehen baut die mechanische Kupplung axial sehr kurz und kommt auch nur mit zwei prinzipiellen Bauelementen aus, nämlich äußerer Ring mit den äußeren Magneten und innerer Ring mit den inneren Magneten, somit sind wenig störanfällige Teile vorhanden, die auf Dauer funktionssicher sind. Mechanische Schocks oder Stöße können aufgrund der Tatsache, dass die Ringe axial unverschieblich sind, nicht zu einer Lösung des magnetischen Kraftschlusses in axialer Richtung führen. Die axiale schmale Bauweise ermöglicht auch durch einfache Baumaßnahmen, die magnetische Abschirmung zu bewerkstelligen. Auch in radialer Richtung baut die magnetische Kupplung nicht ausladend, da die beiden in etwa in einer Ebene angeordneten Ringe wenig ausladende Bauelemente bilden.

In einer weiteren Ausgestaltung der Erfindung weist der äußere Ring außen eine ferromagnetische Abschirmung auf, um störende, nach außen gerichtete magnetische Streufelder abzuschirmen.

Diese Maßnahme hat den Vorteil, dass durch die äußere Abschirmung sichergestellt ist, dass von der magnetischen Kupplung keine störenden, nach außen gerichteten magnetischen Streufelder ausgehen.

In einer weiteren Ausgestaltung der Erfindung besteht der äußere Ring aus ferromagnetischem Material, an dessen Innenseite der zumindest eine Magnet angebracht ist.

Diese Maßnahme hat nun den Vorteil, dass die Abschirmung zugleich das Trägerelement bzw. den eigentlichen Ring ausmacht, an dessen Innenseite der zumindest eine Magnet angebracht ist.

Dies führt ebenfalls zu baulich einfachen, wenig ausladend bauenden Teilen.

In einer weiteren Ausgestaltung der Erfindung sind umfänglich verteilt eine Vielzahl an radial polarisierten Magneten an der Innenseite des äußeren Ringes angeordnet.

Durch die Anordnung von einer Vielzahl an radial polarisierten Magneten kann der Ring in axialer Richtung äußerst schmal ausgebildet werden, da durch die Vielzahl der Magnete der notwendige magnetische Kraftschluss mit dem inneren Magnet in jeder Drehstellung ausgebildet werden kann.

In einer weiteren Ausgestaltung der Erfindung ist der äußere Ring als Stellring ausgebildet.

Diese Maßnahme hat den Vorteil, dass der äußere Ring auch zugleich als Stellring fungiert, dieser also von der Außenseite ergriffen werden kann, somit besonders wenige Bauteile notwendig sind, was zu einer auch in radialer Richtung schlanken Bauweise führt.

In einer weiteren Ausgestaltung der Erfindung ist der äußere Ring außen mit einem Stellring verbunden.

Diese Maßnahme hat nun den Vorteil, dass das Material von äußerem Ring und diesen umgebenden Stellring frei ausgewählt werden kann, so dass auch Designansprüchen Rechnung getragen werden kann, wenn sich bspw. die äußere Gestaltung des Stellringes an die weitere äußere Ausgestaltung des endoskopischen Systems anpassen soll. Es ist auch möglich, den Stellring zugleich als Abschirmung auszubilden und den inneren eigentlichen Ring bspw. aus Kunststoff auszubilden, in den dann die Magnete eingegossen sind. Der äußere Ring kann manuell verdreht oder über einen motorischen, insbesondere elektromotorischen Antrieb bewegt werden.

In einer weiteren Ausgestaltung der Erfindung sind auf der äußeren Seite des inneren Ringes umfänglich verteilt eine Vielzahl an radial polarisierten Magneten angeordnet.

Diese Maßnahme hat den erheblichen Vorteil, insbesondere mit der zuvor erwähnten Maßnahme der Vielzahl an Magneten an der Innenseite des äußeren Ringes, dass mit extrem axial schmal bauenden Ringen ein ausreichender Kraftschluss geschaffen werden kann, um auch relativ schwere oder schwergängige Bauelemente zu bewegen. Dies trägt ferner zu einer wenig ausladenden, also sowohl in axialer als auch in radialer Richtung schlanken Bauweise bei.

In einer weiteren Ausgestaltung der Erfindung sind Anzahl, umfängliche Verteilung und axiale Ausdehnung der Magnete von innerem Ring und äußerem Ring gleich.

Diese Maßnahme hat den erheblichen Vorteil, dass sich zwischen den einzelnen identischen gegenüberstehenden Magneten ein intensiver magnetischer Fluss ausbildet, so dass auch Relativverschiebungen zwischen äußerem und innerem Ring bei ungeschicktem oder rückartigem Bewegen des äußeren Rings nicht stattfinden. Dies trägt erheblich zur Betriebssicherheit bei.

In einer weiteren Ausgestaltung der Erfindung ist der Verdrehbereich des äußeren Ringes begrenzt.

Diese an sich bekannte Maßnahme hat den Vorteil, dass kein Überdrehen möglich ist.

In einer weiteren Ausgestaltung der Erfindung sind die Magnete als Sm-Co-Magnete ausgeführt, welche vorzugsweise eine Legierung aus SmCo₅ und/oder Sm₂Co₁₇ auf Basis von Eisenmetall aufweisen.

Diese Maßnahme hat den Vorteil, dass solche Samarium-Cobalt-Magnete eine hohe Temperaturbeständigkeit bis 200°C aufweisen. Die extremen Temperaturschwankungen, denen endoskopische Systeme beim Autoklavieren ausgesetzt sind, beeinträchtigen die Magnetisierung nicht.

Es versteht sich, dass die vorstehend genannten und die nachstehend noch zu erläuternden Merkmale nicht nur in den jeweils angegebenen Kombinationen, sondern auch in anderen Kombinationen oder in Alleinstellung verwendbar sind, ohne den Rahmen der vorliegenden Erfindung zu verlassen.

Die Erfindung wird nachfolgend anhand einiger ausgewählter Ausführungsbeispiele in Zusammenhang mit den beiliegenden Zeichnungen näher beschrieben und erläutert. Es zeigen:
- Fig. 1: einen Längsschnitt einer Vorrichtung zum axialen Positionieren von optischen Bauelementen,
- Fig. 2: einen Schnitt längs der Linie II-II in Fig. 1,
- Fig. 3: einen Schnitt längs der Linie III-III in Fig. 1, und
- Fig. 4: einen Querschnitt eines Ausführungsbeispiels einer erfindungsgemäßen Vorrichtung zum umfänglichen Positionieren bzw. Ein- und Ausschwenken von Bauelementen in ein optisches System.

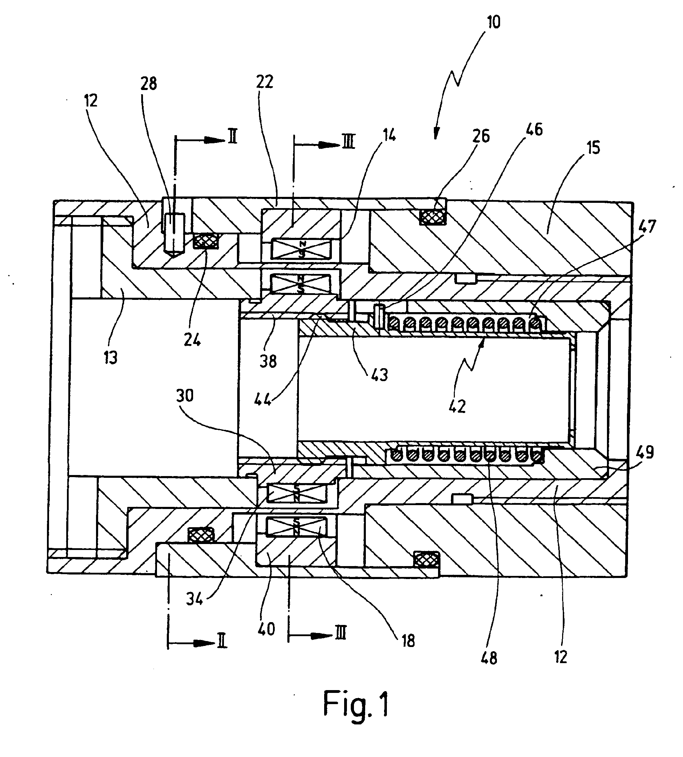

Eine in den Fig. 1 bis 3 dargestellte Vorrichtung ist in ihrer Gesamtheit mit der Bezugsziffer 10 bezeichnet.

Die Vorrichtung 10 ist Teil eines Optikkopfes eines Endoskopes, wobei sich in der Darstellung von Fig. 1 an der rechten Seite eine hier der Übersichtlichkeit nicht dargestellte Okularmuschel anschließt, an der linken Seite der Darstellung von Fig. 1 ist der Schaft des Endoskopes angebracht.

Die Vorrichtung 10 weist ein grob hohlzylindrisches Gehäuse 12 auf, das umfänglich aus einem Stück besteht, und somit hermetisch abgeschlossen ist.

An den Stirnseiten des Gehäuses 12 bilden die zuvor erwähnten Bauelemente, also Okularmuschel einerseits und Endoskopschaft andererseits, einen entsprechenden dichtenden Abschluss.

Eine innere Montagehülse 13 und eine äußere Montagehülse 15 dienen als Montageelemente für die im Gehäuse 12 aufgenommenen, nachfolgend noch zu beschreibenden Elemente.

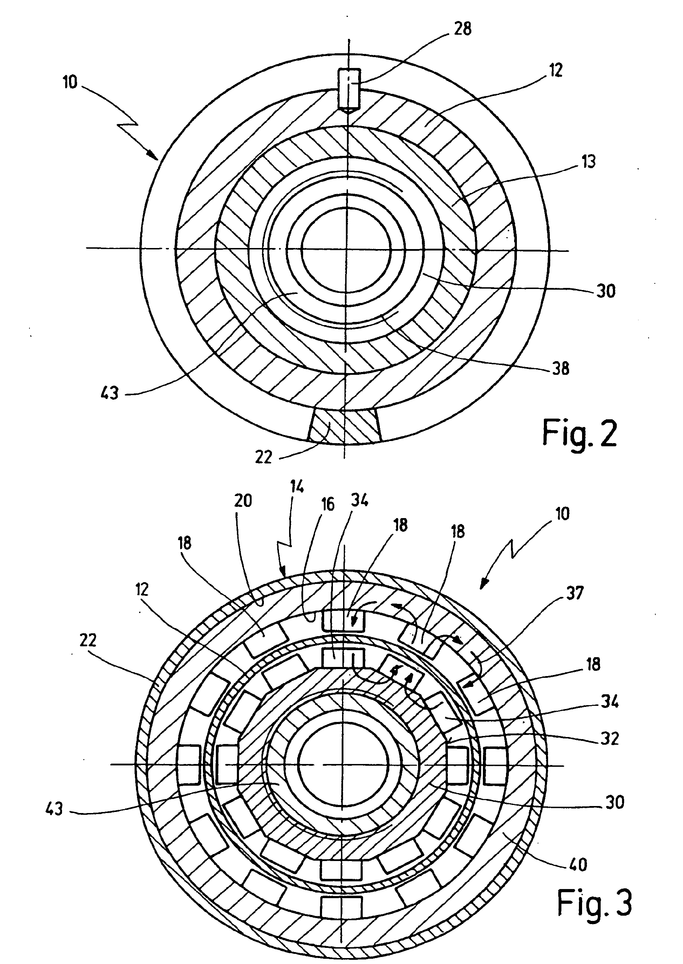

Über die Außenseite des Gehäuses 12 ist ein äußerer Ring 14 geschoben, dessen Innenseite 16 umfänglich gleichmäßig verteilt mit zwölf radial polarisierten Rundmagneten 18 versehen ist. Wie insbesondere aus der Schnittdarstellung von Fig. 3 zu erkennen, kommt die innere Seite der Magnete 18 in einem geringfügigen Abstand zur Außenseite des Gehäuses 12 zum Liegen.

Auf der Außenseite 20 des äußeren Ringes 14 ist ein Stellring 22 montiert, der axial wesentlich länger als der äußere Ring 14 ist und der über O-Ringdichtungen 24 und 26 dichtend, jedoch verdrehbar um die Außenseite des Gehäuses 12 angebracht ist. Ein radial vorspringender Anschlag 28 im Gehäuse 12 begrenzt die umfängliche Verdrehbarkeit des äußeren Ringes 14, wie das insbesondere aus Fig. 2 ersichtlich ist.

Im Innern des Gehäuses 12 ist ein innerer Ring 30 montiert, dessen äußere Seite 32 ebenfalls mit zwölf Rundmagneten 34 versehen ist.

Wie das insbesondere aus der Schnittdarstellung von Fig. 3 ersichtlich ist, ist die umfängliche Verteilung und Anordnung der Magnete 34 so, dass jeweils ein Magnet 34 direkt einem entsprechenden Magnet 18 gegenüberliegt. Die Außenseite der Magnete 34 erstreckt sich bis nahezu an die Innenseite des Gehäuses 12 heran.

Die Magnete sind somit nur in einem äußerst geringfügigen radialen Abstand zueinander angeordnet, jedoch durch das hermetisch abgedichtete Gehäuse 12 voneinander getrennt. Die Magnete 34 sind ebenfalls radial polarisiert und deren Polarität ist so ausgerichtet (siehe Fig. 1 und Fig. 3), dass ein Magnetfluss stattfindet, wie er in Fig. 3 durch die Linien 37 angedeutet ist. Die Magnete 18 und 34 sind als Sm-Co-Magnete ausgeführt.

In dem dargestellten Ausführungsbeispiel besteht der äußere Ring 14 aus einem ferromagnetischen Material, bildet somit eine Abschirmung 40 gegenüber der Außenseite. Das heißt, es treten über die Außenseite keine Magnetfelder hinaus. Das ferromagnetische Material bildet gegenüber der Außenseite, also der Luft, einen wesentlich geringeren magnetischen Widerstand, so dass nahezu die Gesamtheit des äußeren Magnetflusses über die Abschirmung 40 läuft.

Im Inneren des Gehäuses 12 ist ein Bauelement 42 angeordnet, das eine Hülse 43 aufweist. In der Hülse 43 sind optische Bauelemente wie bspw. Linsen angeordnet, die der Übersichtlichkeit halber nicht dargestellt sind.

Die Außenseite der Hülse 43 ist mit einer radial vorspringenden Nase 44 versehen, die in einen Gewindegang 38 an der Innenseite 36 des inneren Rings 30 eingreift.

Ein von der Außenseite 47 der Hülse 43 radial vorstehender Stift 46 dient als Verdrehsicherung, er greift in eine entsprechend hier nicht näher bezeichnete Axialnut in eine die Hülse 43 umgebende Außenhülse 49 ein.

In dem Raum zwischen Außenhülse 49 und Hülse 43 ist eine vorgespannte schraubenlinienförmige Feder 48 angeordnet, die die axial verschiebliche Hülse 43 gegenüber der ortsfesten Außenhülse 49 axial mit Druck beaufschlagt.

Wird nunmehr der äußere Ring 14 über den Stellring 22 verdreht, wird auch der innere Ring 30 aufgrund des Kraftschlusses zwischen den Magneten 18 und 34 verdreht. Diese Verdrehbewegung wird über den Gewindegang 28 und die Nase 44 in eine axiale Verschiebung der Hülse 43 umgesetzt.

Dadurch können die in der Hülse 43 aufgenommenen optischen Bauelemente gegenüber weiteren im Optikkopf aufgenommenen Bauelementen verschoben, bspw. justiert oder fokussiert werden.

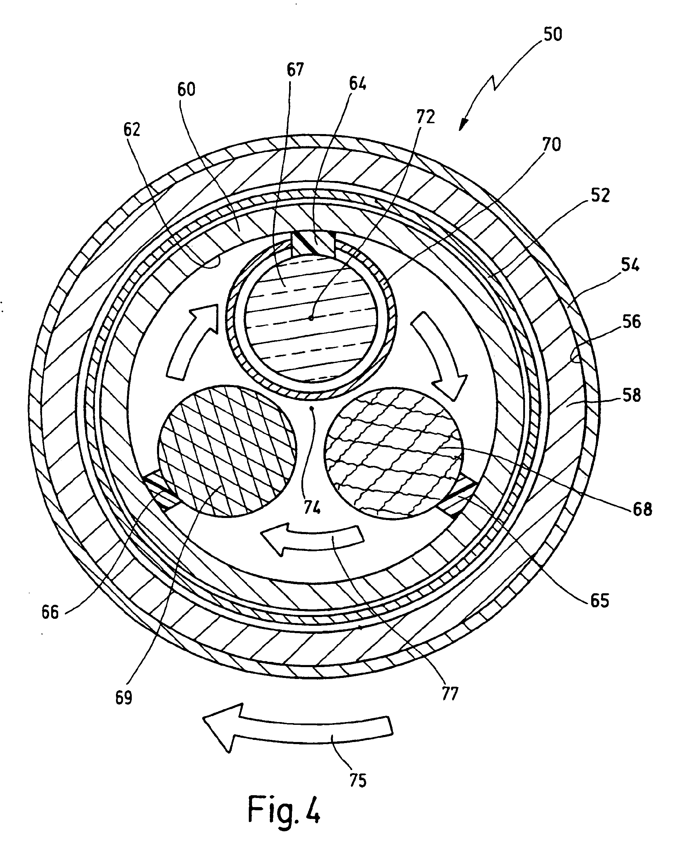

Bei einem in Fig. 4 dargestellten Ausführungsbeispiel einer erfindungsgemäßen Vorrichtung 50 ist ebenfalls ein etwa zylindrisches hermetisch abgeschlossenes Gehäuse 52 vorgesehen, das stirnseitig durch weitere Bauelemente abgeschlossen ist. Um das Gehäuse 52 ist ein äußerer Ring 54 angeordnet, an dessen Innenseite 56 ein Ringmagnet 58 angeordnet ist.

Dementsprechend ist im Gehäuse 52 ein innerer Ring 60 angeordnet, der selbst als Ringmagnet ausgebildet ist.

An der Innenseite des inneren Ringes 60 stehen umfänglich um 120° verteilt drei radial vorspringende Träger 64, 65, 66 vor, die jeweils Bauelemente 67, 68, 69 tragen.

Das Bauelement 67 ist bspw. eine Linse, das Bauelement 68 ein Filter, das Bauelement 69 ein Polarisator.

Im Innenraum des Gehäuses 52 ist ein optisches System 70 angeordnet, dessen optische Achse 72 abseits der Mittellängsachse 74 des zylindrischen Gehäuses 52 liegt.

Die hier nicht näher bezeichneten Mittelpunkte der etwa kreisförmigen Bauelementen 67, 69, 69 liegen so, dass sie auf einem Umfangskreis zum Liegen kommen, dessen Radius dem Abstand zwischen Mittellängsachse 74 und optischer Achse 72 entspricht.

Wird nun der äußere Ring 54, der als Stellring ausgebildet sein kann oder auch die zuvor beschriebene Abschirmung noch zusätzlich aufweisen kann, verdreht, wie das durch einen Pfeil 75 dargestellt ist, wird dann auch dementsprechend der innere Ring 60 verdreht, wie das durch die Pfeile 77 dargestellt ist.

In der in Fig. 4 dargestellten Drehstellung wurde gerade das optische Bauelement 70 genau in den optischen Pfad des optischen Systems 70 verschwenkt.

Durch weiteres Drehen des äußeren Ringes 54 um 120° in Uhrzeigerrichtung wird dann das Bauelement 67 aus dem optischen System 70 herausgeschwenkt und dafür das Bauelement 69 eingeschwenkt. Durch Drehen in entgegengesetzter Richtung oder weiteres Drehen um 120° wird dann dementsprechend das Bauelement 68 eingeschwenkt.

Es ist selbstverständlich möglich, dass das optische System 70 an einer anderen axialen Stelle mit der zuvor in Zusammenhang mit Fig. 1 und 3 beschriebenen Vorrichtung ausgestattet ist, um bspw. Fokussier- oder Justiervorgänge durchzuführen, und außerdem die Vorrichtung 50 zum Ein- bzw. Ausschwenken der weiteren Systeme aufweist. Aufgrund der axial sehr kurzen Bauweise können somit sowohl eine Vorrichtung 10 als auch eine Vorrichtung 50 an ein und demselben optischen Instrument vorhanden sein.

Bei der Ausführung in Zusammenhang mit Fig. 4 liegen die Bauelementen 67, 68, 69 in etwa einer Ebene.

Es ist auch möglich, diese axial versetzt anzuordnen, wobei diese dann an der Innenseite eines Bauelementes angeordnet sind, wie das in Fig. 1 die Hülse 43 darstellt. Dann verursacht ein Verdrehen des äußeren Ringes 54 ein umfängliches Verdrehen und gleichzeitig axiales Bewegen der in das optische System 70 ein- bzw. auszuschwenkenden Elemente. Dies wird dann der Fall sein, wenn radial ausladende Bauelemente eingeschwenkt werden sollen, diese also nicht, wie in Fig. 4 dargestellt, in einer Ebene Platz zur Verfügung haben.

## Patentansprüche

1. Vorrichtung zum Positionieren von Bauelementen (67, 68, 69) innerhalb endoskopischer Systeme, mit einem hermetisch dichten Gehäuse (52), mit einem um die Außenseite des Gehäuses (52) drehbar angeordneten äußeren Ringelement, das umfänglich zumindest einen Magneten (58) trägt, mit einem weiteren, innerhalb des dichten Gehäuses (52) angeordneten inneren Ringelement, das ebenfalls zumindest einen Magneten trägt, wobei die Magnete (58) so ausgerichtet sind, dass durch eine Drehbewegung des äußeren Ringelementes über magnetischen Kraftschluss der zumindest eine innere Magnet bewegt wird, wobei diese Bewegung zum Positionieren von Bauelementen (67, 68, 69) dient, **dadurch gekennzeichnet, dass** sowohl das äußere als auch das innere Ringelement als drehbare, jedoch axial unverschieblich angeordnete Ringe (54, 60) ausgebildet sind, die gegenüberliegend die Magnete (58) tragen, und dass der innere Ring (60) mit den zu positionierenden Bauelementen (67, 68, 69) mechanisch in Verbindung steht, wobei der innere Ring (60) Bauelemente (67, 68, 69) trägt, die bei einem Verdrehen des inneren Ringes (60) in ein optisches System (70) ein- bzw. ausschwenkbar sind.

2. Vorrichtung nach Anspruch 1, **dadurch gekennzeichnet, dass** der äußere Ring außen eine ferromagnetische Abschirmung aufweist, um störende, nach außen gerichtete magnetische Streufelder abzuschirmen.

3. Vorrichtung nach Anspruch 2, **dadurch gekennzeichnet, dass** der äußere Ring aus ferromagnetischem Material besteht, an dessen Innenseite der zumindest eine Magnet angebracht ist.

4. Vorrichtung nach einem der Ansprüche 1 bis 3, **dadurch gekennzeichnet, dass** umfänglich verteilt eine Vielzahl an radial polarisierten Magneten an der Innenseite des äußeren Ringes angeordnet ist.

5. Vorrichtung nach einem der Ansprüche 1 bis 4, **dadurch gekennzeichnet, dass** der äußere Ring (54) als Stellring ausgebildet ist.

6. Vorrichtung nach einem der Ansprüche 1 bis 5, **dadurch gekennzeichnet, dass** der äußere Ring außen mit einem Stellring verbunden ist.

7. Vorrichtung nach einem der Ansprüche 1 bis 6, **dadurch gekennzeichnet, dass** auf der äußeren Seite des inneren Ringes umfänglich verteilt eine Vielzahl an radial polarisierten Magneten angeordnet ist.

8. Vorrichtung nach Anspruch 7, **dadurch gekennzeichnet, dass** Anzahl, umfängliche Verteilung und axiale Ausdehnung der Magnete von innerem Ring (60) und äußerem Ring (54) gleich sind.

9. Vorrichtung nach einem der Ansprüche 1 bis 8, **dadurch gekennzeichnet, dass** der Verdrehbereich des äußeren Ringes (54) begrenzt ist.

10. Vorrichtung nach einem der Ansprüche 1 bis 9, **dadurch gekennzeichnet, dass** die Magnete als Sm-Co-Magnete ausgeführt sind, welche vorzugsweise eine Legierung aus SmCo₅ und/oder Sm₂Co₁₇ auf Basis von Eisenmetall aufweisen.

## Claims

1. A device for positioning components (67, 68, 69) within endoscopic systems, comprising a hermetically sealed housing (52), an outer ring element mounted to rotate about the outside of the housing (52) and carrying on its circumference at least one magnet (58), another inner ring element mounted inside the sealed housing (52) and likewise carrying at least one magnet, the magnets (58) being aligned in such a way that any rotation of the outer ring element has the effect to move the at least one inner magnet by magnetic coupling, such movement serves for positioning components (67, 68, 69), **characterized in that** both, the outer and the inner ring elements, are configured as rotatable, but axially non displaceable rings (54, 60) carrying said oppositely arranged magnets (58), and that said inner ring (60) is mechanically connected with the components (67, 68, 69) to be positioned, wherein the inner ring (60) carries components (67, 68, 69) that can be swung into and onto of an optical system by rotation of the inner ring (60).

2. The device of claim 1, **characterized in that** said outer ring is provided on its outside with a ferromagnetic screening in order to screen any interfering, outwardly directed magnetic stray fields.

3. The device of claim 2, **characterized in that** said outer ring is made from a ferromagnetic material with said at least one magnet mounted on its inside.

4. The device of anyone of claims 1 to 3, **characterized in that** a plurality of radially polarized magnets are mounted on the inside of said outer ring, in circumferentially distributed arrangement.

5. The device of anyone of claims 1 to 4, **characterized in that** said outer ring (54) is configured as a setting ring.

6. The device of anyone of claims 1 to 5, **characterized in that** said outer ring is connected on its outside with a setting ring.

7. The device of anyone of claims 1 to 6, **characterized in that** a plurality of radially polarized magnets are provided on the outside of said inner ring in circumferentially distributed arrangement.

8. The device of claim 7, **characterized in that** the number, circumferential distribution and axial extension of the magnets are equal for said inner (60) and said outer ring (54).

9. The device of anyone of claims 1 to 8, **characterized in that** the range of rotation of said outer ring (54) is limited.

10. The device of anyone of claims 1 to 9, **characterized in that** the magnets are designed as Sm-Co magnets, comprising preferably an alloy of SmCo₅ and/or Sm₂Co₁₇ based on ferrous metal.

## Revendications

1. Dispositif pour le positionnement de composants (67, 68, 69) à l'intérieur de systèmes endoscopiques, avec un boîtier hermétique étanche (52), avec un élément annulaire extérieur rotatif disposé autour de la face extérieure du boîtier (52) supportant au moins un aimant (58) sur sa circonférence, avec un autre élément annulaire intérieur disposé à l'intérieur du boîtier hermétique (52), lequel supporte également un aimant au moins, les aimants (58) étant orientés de telle manière qu'un aimant intérieur au moins soit mû par une rotation de l'élément annulaire extérieur du fait de la force d'adhérence magnétique, ce mouvement servant au positionnement de composants (67, 68, 69), **caractérisé en ce que** l'élément annulaire extérieur aussi bien que l'élément annulaire intérieur sont configurés comme bagues (54, 60) rotatives quoique axialement fixes, lesquelles supportent des aimants (58) se faisant face, et **en ce que** la bague intérieure (60) est mécaniquement reliée aux composants à positionner (67, 68, 69), ladite bague intérieure (60) supportant des composants (67, 68, 69) pivotant dans un sens ou dans l'autre lors d'une rotation de la bague intérieure (60) dans un système optique (70).

2. Dispositif selon la revendication 1, **caractérisé en ce que** la bague extérieure présente un blindage ferromagnétique extérieur, pour l'inhibition de champs magnétiques de dispersion perturbateurs dirigés vers l'extérieur.

3. Dispositif selon la revendication 2, **caractérisé en ce que** la bague extérieure est en matériau ferromagnétique, sur la face intérieure duquel un aimant au moins est appliqué.

4. Dispositif selon l'une des revendications 1 à 3, **caractérisé en ce que** plusieurs aimants radialement polarisés sont disposés sur la face intérieure de la bague extérieure, circonférentiellement répartis.

5. Dispositif selon l'une des revendications 1 à 4, **caractérisé en ce que** la bague extérieure (54) est formée comme bague de réglage.

6. Dispositif selon l'une des revendications 1 à 5, **caractérisé en ce que** la bague extérieure est extérieurement reliée à une bague de réglage.

7. Dispositif selon l'une des revendications 1 à 6, **caractérisé en ce que** plusieurs aimants radialement polarisés sont disposés sur la face extérieure de la bague intérieure, circonférentiellement répartis.

8. Dispositif selon la revendication 7, **caractérisé en ce que** le nombre, la répartition circonférentielle et l'extension axiale des aimants sont identiques pour la bague intérieure (60) et pour la bague extérieure (54).

9. Dispositif selon l'une des revendications 1 à 8, **caractérisé en ce que** la plage de rotation de la bague extérieure (54) est limitée.

10. Dispositif selon l'une des revendications 1 à 9, **caractérisé en ce que** les aimants sont exécutés comme aimants Sm-Co, lesquels présentent préférentiellement un alliage de SmCo₅ et/ou de Sm₂Co₁₇ à base de métal ferreux.
